# EUROPEAN PATENT APPLICATION

(11) **EP 1 952 824 A1**
(43) Date of publication of application: **06.08.2008**
(21) Application number: 08153319.2
(22) Date of filing: 19.11.2004
(51) Int. Cl.: A61K 45/06, A61K 31/4422, A61K 31/445, A61P 25/28

(54) **Compositions and methods of treatment using L-type calcium channel blockers and cholinesterase inhibitors**

(30) Priority: 21.11.2003 US 523664 P; 09.09.2004 US 608116 P
(62) Divisional of application: 04811351.8
(71) Applicant: Memory Pharmaceuticals Corporation, Montvale, NJ 07645 (US)
(72) Inventor: Murphy, Christa, Ramsey, New Jersey 07446 (US); Rose, Gregory M., Guilford, CT 06437 (US); Unterbeck, Axel, Madison, CT 06443-0662 (US)
(74) Representative: Vossius & Partner

(57) **Abstract**

The present invention relates to compositions at least one L-type calcium channel blocker, particularly the compound (+)-isopropyl 2-methoxyethyl 4-(2-chloro-3-cyano-phenyl)-1,4-dihydro-2,6-dimethyl-pyridine-3,5-dicarboxylate, in combination with at least one cholinesterase inhibitor, particularly donepezil, and uses thereof in methods of treatment.

## Description

This application claims the benefit of Serial No. 60/523,664, filed November 21, 2003, and Serial No. 60/608,116, filed September 9, 2004.

The present invention relates to compositions comprising at least one L-type calcium channel blocker, particularly the compound (+)-isopropyl 2-methoxyethyl 4-(2-chloro-3-cyano-phenyl)-1,4-dihydro-2,6-dimethyl-pyridine-3,5-dicarboxylate, in combination with at least one cholinesterase inhibitor, particularly donepezil, and uses thereof in methods of treatment.

### BACKGROUND OF THE INVENTION

Meier at al. (US 5,665,740), the entire disclosure of which is hereby incorporated by reference, disclose that the compound, (+)-isopropyl 2-methoxyethyl 4-(2-chlara-3-cyano-phenyl)-1,4-dihydro-2,6-dimethyl-pyridine-3,5-dicarboxylate, has a positive effect on learning and memory powers and has antidepressant potential.

The condition of memory impairment is manifested by impairment of the ability to leam new information and/or the inability to recall previously learned information. Memory impairment is a primary symptom of dementia and can also be a symptom associated with a variety of diseases and conditions such as Alzheimer's disease or age-related cognitive decline.

The present invention relates to compositions containing at least one L-type calcium channel blockers such as (+)-isopropyl 2-methoxyethyl 4-(2-chlora-3-cyanaphenyl)-1,4-dihydro-2,6-dimethyl-pyridine-3,5-dicarboxylate in combination with at least one cholinesterase inhibitor, particularly donepezil, and uses thereof in methods of treatment, particularly treatments for memory and/or cognitive impairment.

### SUMMARY OF THE INVENTION

The present invention includes a method of treating memory and/or cognitive impairment comprising administering to a patient (e.g., a human), simultaneously or sequentially, an L-type calcium channel blocker and a cholinesterase inhibitor. In methods using simultaneous administration, the agents can be present in a combined composition or can be administered separately. According to an embodiment of this aspect of the invention, the L-type calcium channel blocker is selected from dihydropyridines having calcium channel blocking activity. According to another embodiment of this aspect of the invention, the L-type calcium channel blocker is amlodipine, felodipine, isradipine, lacidipine, lercanidipine, nicardipine, nifedipine, nimodipine, nitrendipine, nisoldipine, or (+) isopropyl 2-methoxyethyl 4-(2-chloro-3-cyano-phenyl)-1,4-dihydro-2,6-dimethylpyridine-3,5-dicarboxylate. According to another embodiment of this aspect of the invention, the cholinesterase inhibitor is donepezil, rivastigmine, galantamine, icopezil, pyridostigmine, edrophonium, neostigmine, physostigmine, Huperzine A, phenserine, or tacrine. According to further embodiment of this aspect of the invention, the L-type calcium channel blocker is amlodipine, felodipine, isradipine, lacidipine, lercanidipine, nicardipine, nifedipine, nimodipine, nitrendipine, nisoldipine, or (+) isopropyl 2-methoxyethyl 4-(2-chloro-3-eyano-phonyl)-1,4-dihydro-2,6-dimethylpyridine-3,5-dicarboxylate, and the cholinesterase inhibitor is donepezil, rivastigmine, galantamine, icopezil, pyridostigmine, edrophonium, neostigmine, physostigmine, Huperzine A, phenserine, or tacrine.

For example, the compound, (+) isopropyl 2-methoxyethyl 4-(2-chloro-3-cyano-pbenyl)-1,4-dihydro-2,6-dimethylpyridine-3,5-dicarboxylate, can be administered in combination with a cholinesterase inhibitors (e.g., donepezil, rivastigmine, galantamine, icopezil, pyridostigmine, edrophonium, neostigmine, physostigmine, Huperzine A, phenserine, or tacrine). In such combinations, each active ingredient can be administered either in accordance with their usual dosage range or a dose below their usual dosage range.

According to a further aspect, the invention includes a method of treating memory and/or cognitive impairment associated with Alzheimer's disease comprising administering to a patient (e.g., a human), simultaneously or sequentially, an L-type calcium channel blocker and a cholinesterase inhibitor. In methods using simultaneous administration, the agents can be present in a combined composition or can be administered separately. According to an embodiment of this aspect of the invention, the L-type calcium channel blocker is selected from dihydropyridines having calcium channel blocking activity. According to another embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydro-2,6-dimethylpyridine-3,5-dicarboxylate. According to another embodiment of this aspect of the invention, the cholinesterase inhibitor is donepezil, rivastigmine, galantamine, icopezil, pyridostigmine, edrophonium, neostigmine, physostigmine, Huperzine A, phenserine, or tacrine. According to further embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydro-2,6-dimethylpyridine-3,5-dicarboxylate and the cholinesterase inhibitor is donepezil, rivastigmine, galantamine, icopezil, pyridostigmine, edrophonium, neostigmine, physostigmine, Huperzine A, phenserine, or tacrine.

For example, the invention includes methods for treating memory and/or cognitive impairment associated with Alzheimer's disease comprising administering to a patient (e.g., a human), simultaneously or sequentially, the compound (+) isopropyl 2-methoxyethyl 4-(2-chloro-3-cyano-phenyl)-1,4-dihydro-2,6-dimethylpyridine-3,5-dicarboxylate and a cholinesterase inhibitor used in the treatment of Alzheimer's disease such as Reminyl (galantamine, specifically galantamine hydrobromide), Cognex (tacrine, specifically tacrine hydrochloride), Aricept (donepezil, specifically donepezil hydrochloride), and Exelon (rivastigmine, specifically rivastigmine tartrate). In methods using simultaneous administration, the agents can be present in a combined composition or can be administered separately.

According to a further aspect, the invention includes a method of treating memory and/or cognitive impairment associated with dementia comprising administering to a patient (e.g., a human), simultaneously or sequentially, an L-type calcium channel blocker and a cholinesterase inhibitor. In methods using simultaneous administration, the agents can be present in a combined composition or can be administered separately. According to an embodiment of this aspect of the invention, the L-type calcium channel blocker is selected from dihydropyridines having calcium channel blocking activity. According to another embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydra-2,6-dimethylpyridine-3,5-dicarboxylate. According to another embodiment of this aspect of the invention, the cholinesterase inhibitor is donepezil,rivastigmine galantamine, icopezil, pyridostigmine, edrophonium, neostigmine, physostigmine, Huperzine A, phenserine, or tacrine. According to further embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyana-phenyl)-1,4-dihydro-2,6-dimethylpyridine-3,5-dicarboxylate and the cholinesterase inhibitor is donepezil, rivastigmine, galantamine, icopezil, pyridostigmine, edrophonium, neostigmine, physostigmine, Huperzine A, phenserine, or tacrine.

For example, the invention includes methods for treating memory and/or cognitive impairment associated with dementia comprising administering to a patient (e.g., a human), simultaneously or sequentially, the compound (+) isopropyl 2-methoxyethyl 4-(2-chloro-3-cyano-phenyl)-1,4-dihydro-2,6-dimethylpyridine-3,5-dicarboxylate and a cholinesterase inhibitor used in the treatment of dementia such as Cognex (tacrine, specifically tacrine hydrochloride), Aricept (donepezil, specifically donepezil hydrochloride), and Exelon (rivastigmine, specifically rivastigmine tartrate). In methods using simultaneous administration, the agents can be present in a combined composition or can be administered separately.

According to a further aspect, the invention includes a method of treating memory and/or cognitive impairment comprising administering to a patient (e.g., a human), simultaneously or sequentially, (+) isopropyl 2-methoxyethyl 4-(2-chloro-3-cyanophenyl)-1,4-dihydro-2,6-dimethylpyridine-3,5-dicarboxylate and donepezil (e.g., (+/-) 2,3-dihydro-5,6-dimethoxy-2-[[1-(phenylmethyl)-4-piperidinyl]methyl]-1H-indene-1-one hydrochloride). In methods using simultaneous administration, the agents can be present in a combined composition or can be administered separately.

According to a further aspect, the invention includes a method of treating memory and/or cognitive impairment associated with Alzheimer's disease comprising administering to a patient (e.g., a human), simultaneously or sequentially, (+) isopropyl 2-methoxyethyl 4-(2-chloro-3-cyano-phenyl)-1,4-dihydro-2,6-dimethylpyridine-3,5-dicarboxylate and donepezil. In methods using simultaneous administration, the agents can be present in a combined composition or can be administered separately.

According to a further aspect, the invention includes a method of treating memory and/or cognitive impairment associated with dementia comprising administering to a patient (e.g., a human), simultaneously or sequentially, (+) isopropyl 2-methoxyethyl 4-(2-chloro-3-cyano-phenyl)-1,4-dihydro-2,6-dimethylpyridine-3,5-dicarboxylate and donepezil. In methods using simultaneous administration, the agents can be present in a combined composition or can be administered separately.

Meier at al. (US 5,665,740) discloses that (+)-isopropyl 2-methoxyethyl 4-(2-chloro-3-cyano-phenyl)-1,4-dihydro-2,6-dimethyl-pyridine-3,5-dicarboxylate can be employed for: the treatment of central degenerative disorders, as, for example, occur in dementias (multi-infarct dementia, MID, primary degenerative dementia PDD, pre- and senile Alzheimer's disease, HIV dementia and other forms of dementia), Parkinson's disease or tropic lateral sclerosis; the treatment of cerebral function disorders in old age, of organic brain syndrome (OBS) and of age-associated memory impairment (AAMI); the prophylaxis and control of the sequelae of cerebral circulatory disorders such as cerebral ischaemias, strokes and of subarachnoid haemorrhages; the treatment of depressions and of mania; the treatment of migraines; the treatment of neuropathies, which are caused e,g. by metabolic disorders such as diabetes mellitus, traumas, intoxifications, microorganisms or autoimmune disorders; the treatment of addictive disorders; and the treatment of withdrawal symptoms.

According to a further aspect of this invention, the combination of an L-type calcium channel blocker and a cholinesterase inhibitor can also be used for the above-mentioned treatments disclosed in US 5,665,740. According to an embodiment of this aspect of the invention, the L-type calcium channel blocker is selected from dihydropyridines having calcium channel blocking activity. According to another embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydro-2,6-dimethylpyridine-3,5-dicarboxylate. According to another embodiment of this aspect of the invention, the cholinesterase inhibitor is donepezil, rivastigmine, galantamine, icopezil, pyridostigmine, edrophonium, neostigmine, physostigmine, Huperzine A, phenserine, or tacrine. According to further embodiment of this aspect of the invention, the L-type calcium channel blocker is amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydra-2,6-dimethylpyidine-3,5-dicarboxylate and the cholinesterase inhibitor is donepezil, rivastigmine, galantamine, icopezil, pyridostigmine, edrophonium, neostigmine, physostigmine, Huperzine A, phenserine, or tacrine. For example, the L-type calcium channel blocker can be (+) isopropyl 2-methoxyethyl 4-(2-chloro-3-cyano-phenyl)-1,4-dihydro-2,6-dimethylpyridine-3,5-dicarboxylate and the cholinesterase inhibitor can be Reminyl (galantamine, specifically galantamine hydrobromide), Cognex (tacrine, specifically tacrine hydrochloride), Aricept (donepezil, specifically donepezil hydrochloride), and Exelon (rivastigmine, specifically rivastigmine tartrate).

The dosages of the compounds of the present invention depend upon a variety of factors including the particular syndrome to be treated, the severity of the symptoms, the route of administration, the frequency of the dosage interval, the particular compound utilized, the efficacy, toxicology profile, pharmacokinetic profile of the compound, and the presence of any deleterious side-effects, among other considerations.

In accordance with the invention, the combined treatment of the L-type calcium channel blocker (+) isopropyl 2-methoxyethyl 4-(2-chloro-3-cyano-phenyl)-1,4-dihydro-2,6-dimethylpyridine-3,5-dicarboxylate with the acetylcholine esterase inhibitor donepezil provides synergistic results with regards to the treatment of memory and/or cognitive impairment. See Example 1 discussed below.

The compound (+) isopropyl 2-methoxyethyl 4-(2-chloro-3-cyano-phenyl)-1,4-dihydro-2,6-dimethylpyridine-3,5-dicarboxylate can be administered in an amount of, for example, 10-360 mg/day (e.g., 30mg, 60mg, or 120 mg BID). Donepezil is generally administered in an amount of 5-20 mg/day (generally the dose of donepezil is titrated up to a level that is tolerated but not exceeding 20 mg/day).

However, as a result of the synergy, in accordance with the inventions, isopropyl 2-methoxyethyl 4-(2-chloro-3-cyano-phenyl)-1,4-dihydro-2,6-dimethylpyridine-3,5-dicarboxylate and donepezil can each be administered in an amount which is within the mentioned general ranges or at lower amounts. Thus, isopropyl 2-methoxyethyl 4-(2-chloro-3-cyano-phenyl)-1,4-dihydro-2,6-dimethylpyridine-3,5-dicarboxylate can be administered at 10-360 mg/day, while donepezil is administered at an amount below 5 mg/day (e.g., 0.25, 0.5, 1.0,1.5,2.0,2.5,3.0,3.5,4.0, or 4.5 mg). Alternatively, isopropyl 2-methoxyethyl 4-(2-chloro-3-cyano-phenyl)-1,4-dihydro-2,6-dimethylpyridine-3,5-dicarboxylate can be administered at an amount below 10 mg/day (e.g., 0.5, 1.0, 1.5, 2.0, 2.5, 3.0, 3.5, 4.0, 4.5, 5.0, 6.0, 7.0, 8.0, or 9.0 mg), while donepezil is administered at 5-20 mg/day. In addition, isopropyl 2-methoxyethyl 4-(2-chloro-3-cyano-phenyl)-1,4-dihydro-2,6-dimethylpyridine-3,5-dicarboxylate can be administered at an amount below 10 mg/day (e.g., 0.5, 1.0, 1.5, 2.0, 2.5, 3.0, 3.5, 4.0, 4.5, 5.0, 6.0, 7.0, 8.0, or 9.0 mg), and donepezil can be administered at an amount below 5 mg/day (e.g., 0.25, 0.5, 1.0, 1.5, 2.0, 2.5, 3.0, 3.5, 4.0, or 4.5 mg). Alternatively, also in accordance with the invention, isopropyl 2-methoxyethyl 4-(2-chloro-3-cyano-phenyl)-1,4-dihydro-2,6-dimethylpyridine-3,5-dicarboxylate can be administered at an amount of 10-360 mg/day while donepezil is administered at 5-20 mg/day.

The weight ratio of the daily administered amount of isopropyl 2-methoxyethyl 4-(2-chloro-3-cyano-phenyl)-1,4-dihydro-2,6-dimethylpyridine-3,5-dicarboxylate to the daily administered amount of donepezil is, for example, 5:1 to 30:1, preferably 15:1 to 25:1, for example, 10:1, 12:1, 14:1, 16:1, 17:1, 18:1, 19:1, 21:1, 22:1, 23:1,24:1, 27:1, and 29:1)

Side effects for donepezil include nausea, diarrhea, insomnia, vomiting, muscle cramps, fatigue and anorexia. Thus, preferably donepezil is preferably administered in an amount of less than 5 mg/day while isopropyl 2-methoxyethyl 4-(2-chloro-3-cyanophenyl)-1,4-dihydro-2,6-dimethylpyridine-3,5-dicarboxylate can preferably be administered at 10-360 mg/day, or below 10 mg.

Donepezil and isopropyl 2-methoxyethyl 4-(2-chloro-3-cyano-phenyl)-1,4-dihydro-2,6-dimethylpyridine-3,5-dicarboxylate can be administered simultaneously or sequentially, in combined or separate compositions. The mode of administration can be any of those previously described. However, oral administration, transdermal administration, and rectal administration, are preferred, especially oral administration.

The compound of the invention can be administered alone or as an active ingredient of a formulation. Thus, the present invention also includes pharmaceutical compositions of the compound of the invention, containing, for example, one or more pharmaceutically acceptable carriers, and/or one or more active agents.

Thus, according to a further aspect, the invention includes a pharmaceutical composition comprising an L-type calcium channel blocker and a cholinesterase inhibitor. For example, the L-type calcium channel blocker can be selected from dihydropyridines having calcium channel blocking activity, e.g., amlodipine, felodipine, isradipine, lacidipine, lercanidipine, nicardipine, nifedipine, nimodipine, nitrendipine, nisoldipine, and (+) isopropyl 2-methoxyethyl 4-(2-chloro-3-cyano-phenyl)-1,4-dihydro-2,6-dimethylpyridine-3,5-dicarboxylate. In addition, the cholinesterase inhibitor can be selected from, for example, is donepezil, rivastigmine, galantamine, icopezil, pyridostigmine, edrophonium, neostigmine, physostigmine, Huperzine A, phenserine, and tacrine.

According to further embodiment of this aspect of the invention, the L-type calcium channel blocker is (+) isopropyl 2-methoxyethyl 4-(2-chloro-3-cyano-phenyl)-1,4-dihydro-2,6-dimethylpyridine-3,5-dicarboxylate, and the cholinesterase inhibitor is donepezil. In the pharmaceutical composition, the weight ratio of (+) isopropyl 2-methoxyethyl 4-(2-chloro-3-cyano-phenyl)-1,4-dihydro-2,6-dimethylpyridine-3,5-dicarboxylate to donepezil is, for example, 5:1 to 30:1, preferably 15:1 1 to 25:1.. The compositions can, for example, contain 10-360 mg of (+) isopropyl 2-methoxyethyl 4-(2-chloro-3-cyano-phenyl)- 1,4-dihydro-2,6-dimethylpyridine-3,5-dicarboxylate and 5-20 mg of donepezil. Alternatively, the compositions can, for example, contain less than 10 mg of(+) isopropyl 2-methoxyethyl 4-(2-chloro-3-cyano-phonyl)-1,4-dihydro-2,6-dimethylpyridine-3,5-dicarboxylate and 5-20 mg of donepezil, or 10-360 mg of(+) isopropyl 2-methoxyethyl 4-(2-chloro-3-cyano-phenyl)-1,4-dihydro-2,6-dimethylpyridine-3,5-dicarboxylate and less than 5 mg of donepezil, or less than 10 mg of(+) isopropyl 2-methoxyethyl 4-(2-chloro-3-cyano-phenyl)-1,4-dihydro-2,6-dimethylpyridine-3,5-dicarboxylate and less than 5 mg of donepezil.

Numerous standard references are available that describe procedures for preparing various formulations suitable for administering the compound according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition).

Administration may be accomplished according to patient needs, for example, orally, nasally, parenterally (subcutaneously, intraveneously, intramuscularly, intrasternally and by infusion), rectally, vaginally, topically and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and excipients known in the art, including but not limited to suspending agents, solubilizers, buffering agents, binders, disintegrants, preservatives, colorants, flavorants, lubricants and the like. Time release capsules, tablets and gels are also advantageous in administering the compounds of the present invention.

Various liquid oral dosage forms can also be used for administering compounds of the inventions, including aqueous and non-aqueous solutions, emulsions, suspensions, syrups, and elixirs. Such dosage forms can also contain suitable inert diluents known in the art such as water and suitable excipients known in the art such as preservatives, wetting agents, sweeteners, flavorants, as well as agents for emulsifying and/or suspending the compounds of the invention. The compounds of the present invention may be injected, for example, intravenously, in the form of an isotonic sterile solution. Other preparations are also possible.

Suppositories for rectal administration of the compounds of the present invention can be prepared by mixing the compound with a suitable excipient such as cocoa butter, salicylates and polyethylene glycols. Formulations for vaginal administration can be in the form of a pessary, tampon, cream, gel, paste, foam, or spray formula containing, in addition to the active ingredient, such suitable carriers as are known in the art.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

The dosages of the compounds of the present invention depend upon a variety of factors including the particular syndrome to be treated, the severity of the symptoms, the route of administration, the frequency of the dosage interval, the particular compound utilized, the efficacy, toxicology profile, pharmacokinetic profile of the compound, and the presence of any deleterious side-effects, among other considerations.

The active compounds should be present in these preparations in a concentration of 0. 1 to 99.5% by weight, preferably of 0.5 to 95% by weight of the total mixture. In general, it has proven advantageous to administer the active compounds in total amounts of about 0.01 to about 50 mg/kg, preferably in total amounts of about 0.1 mg/kg to 10 mg/kg of body weight every 24 hours, if appropriate in the form of several individual doses, to achieve the desired result.

One of ordinary skill in the art will recognize that the compound, isopropyl 2-methoxyethyl 4-(2-chloro-3-cyano-phenyl)-1,4-dihydro-2,6-dimethylpyridine-3,5-dicarboxylate, possesses an asymmetric carbon atom and thus is capable of existing in the form of optical isomers, as well as in the form of racemic or nonracemic mixtures thereof. All of these compounds, including racemates, nonracemic mixtures of enantiomers, substantially pure, and pure enantiomers, are within the scope of the present invention. Substantially pure enantiomers contain no more than 5% w/w of the corresponding opposite enantiomer, preferably no more than 2%, most preferably no more than 1%.

The racemic compounds can be synthesized by various procedures, for example, as described in US 5,665,740. For example, 2-chloro-3-cyanobenzaldehyde can be reacted with 2-methoxyethyl acetoacetate to obtain 2-methoxyethyl 2-acetyl-3-(2-chloro-3-cyano)-2-propenoate. This compound is then further reacted with isopropyl amino-2-butenoate to obtain racemic isopropyl 2-methoxyethyl 4-(2-chloro-3-cyano-phenyl)-1,4-dihydro-2,b-dirnethyl-pyridine-3,5-dicarboxylate. (See Examples I and 1 of US 5,665,740.) (+)-isopropyl 2-methoxyethyl 4-(2-chlaro-3-cyano-phenyl)-1,4-dihydro-2,6-dimethyl-pyridine-3,5-dicarboxylate can be obtained by subjecting the racemate to chiral chromatography. (See Example 2 of US 5,665,740.)

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical and/or chemical differences by methods known to those skilled in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of the invention can likewise be obtained by utilizing optically active starting materials in chiral syntheses processes under reaction conditions that do not cause racemization.

The optical isomer can also be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical and/or chemical differences by methods known to those skilled in the art, for example, by chromatography or fractional crystallization, The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compound of the invention can likewise be obtained by utilizing optically active starting materials in chiral syntheses processes under reaction conditions that do not cause racemization.

In addition, one of ordinary skill in the art will recognize that the compounds can be used in different enriched isotopic forms, e.g., enriched in the content of ²H, ³H, ¹¹C, ¹³C and/or ¹⁴C. In one particular embodiment, the compounds are deuterated. Such deuterated forms can be made by the procedures described in U.S. Patent Nos. 5,846,514 and 6,334,997, both of which are hereby incorporated by reference. As described in U.S. Patent Nos. 5,846,514 and 6,334,997, deuteration can improve the efficacy and increase the duration of action of drugs.

Deuterium substituted compounds can be synthesized using various methods such as described in: Dean, Dennis C.; Editor. Recent Advances in the Synthesis and Applications of Radiolabeled Compounds for Drug Discovery and Development. [In: Curr., Pharm. Des., 2000; 6(10)] (2000), 110 pp. CAN 133:68895 AN 2000:473538 CAPLUS; Kabalka, George W.; Varma, Rajender S. The synthesis of radiolabeled compounds VIA organometallic intermediates. Tetrahedron (1989), 45(21), 6601-21, CODEN: TETRAB ISSN:0040-4020. CAN 112:20527 AN 1990:20527 CAPLUS; and Evans, E. Anthony. Synthesis of radiolabeled compounds, J. Radioanal. Chem. (1981), 64(1-2), 9-32. CODEN: JRACBN ISSN:0022-4081, CAN 95:76229 AN 1981:476229 CAPLUS, each of which is hereby incorporated by reference.

In the foregoing and in the following examples, all temperatures are set forth uncorrected in degrees Celsius; and, unless otherwise indicated, all parts and percentages are by weight.

### BRIEF DESCRIPTION OF THE DRAWINGS

Various other features and attendant advantages of the present invention will be more fully appreciated when considered in conjunction with the accompanying drawings, wherein:
Figure 1 illustrates dose response data for Compound A, (+) isopropyl 2- methoxyethyl 4-(2-chloro-3-cyano-phenyl)-1,4-dihydro-2,6-dimethylpyridine-3,5-dicarboxylate, in the Novel Object Recognition Memory test;
Figure 2 illustrates dose response data for donepezil in the Novel Object Recognition Memory test;
Figure 3 presents comparison data for Compound A alone, donepezil alone, memantine alone, the combination of memantine and donepezil and the combination of Compound A and donepezil in the Novel Object Recognition Memory test; and
Figure 4 illustrates dose response data for memantine in the Novel Object Recognition Memory test.

### EXAMPLES

### EXAMPLE 1

Evaluation of Co-administering Donepezil and (+) Isopropyl 2-methoxyethyl 4-(2-chloro-3 -cyano-phenyl)-1,4-dihydro-2,6-dimethylpyridine-3,5-dicarboxylate in Novel Object Recognition Memory

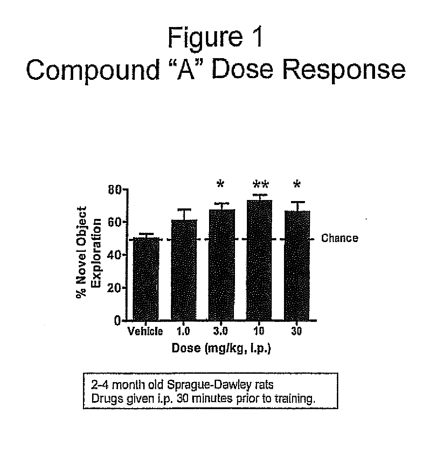

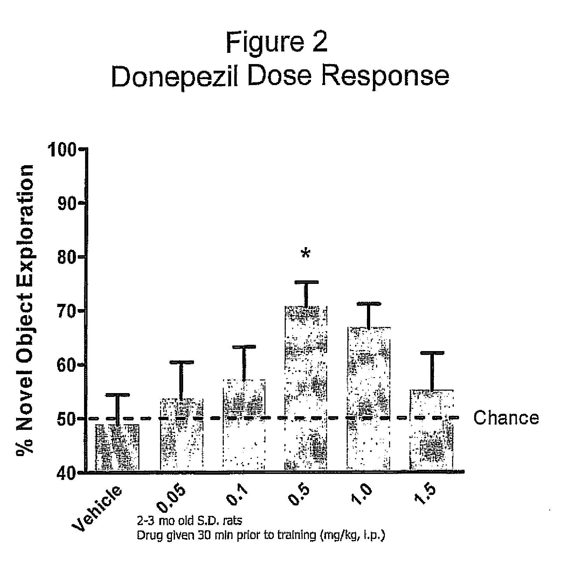

The general procedure for compound evaluation in Novel Object Recognition Memory (Hippocampus-Dependent Nonspatial Memory Task) is as follows:
1. The animals are transported in their home cages from the vivarium to a dimly lit (5-6 lux) training room and acclimated for approximately 45 minutes.
2. The animals are than individually placed in the training/testing environment (an opaque plastic chamber, 61 cm x 42 cm x 37 cm with bedding on the floor) for 5 minutes of habituation. Thereafter, the animals are returned to their home cage. They remained in the testing room for approximately 15 minutes after the last animal is habituated before being returned to their colony room.
3. For the memory test, the objects to be discriminated are a goblet (11 cm in height, 6.5 cm in diameter) and a conical candlestick holder (11 cm in height, 6 cm in diameter) made of green, nontransparent glass. There are multiple copies of each object used for training and testing. Object pairs are randomly assigned within and between conditions. For training, two identical objects are placed 8 cm from the sides of the two short walls of the chamber.
4. The day after initial habituation, the animals are returned to the testing room and acclimated as described in Step 1. Drug and/or vehicle is then administered according to experimental design (i.p.: 26 3/8-gauge needle; or p.o.: 18-gauge needle) at an interval ranging from 24 hours prior to 24 hours post-training. The training procedure is begun by placing the animal, nose facing the wall, into the empty chamber at a position in the center of a long wall. After 1 minute of re-habituation, the animal is removed from the chamber and placed in a holding cage (45 cm x 26 cm x 20 cm) with bedding for a period of 10 seconds. During this delay, the training objects are placed in the chamber. The animal is then returned to the chamber for a 15-minute period of exploration. The animal's movements are observed via cameral located over the chamber and are recorded on videotape. After the training session, the animal is returned to its home cage. The objects are cleaned with an 85% EtOH solution. The animals remain in the testing room for approximately 15 minutes after the last animal is trained before being returned to their colony room.
5. Following a delay interval (1 hour to several weeks, but usually 24 hours), the animals are again returned to the testing room and acclimated as described in Step 1. After this, individual animals are re-habituated to the empty chamber for 1 minute as was described in Step 4. For the test, two new objects are placed in the chamber: one is identical to the objects used during training, while the other is novel. The position of the novel object is randomized for each animal. The animals are allowed to actively explore until they accumulate a total of 60 seconds of exploration. During the test period, the amount of time spent exploring either the novel or the familiar object is recorded using R.E. Clark's V.P.C. program, which also signals when 60 seconds of exploration has been accumulated. The animal's movements are observed via a camera located over the chamber and are recorded on videotape. Any animal that fails to explore either object or to reach the set criterion within 15 minutes is excluded from analysis. After the test, the animals are returned to their home cage, and then to the colony room. The objects are cleaned with an 85% EtOH solution.
6. The dose response data for Compound A and donepezil in the Novel Object Recognition Memory test are shown in Figures 1 and 2, respectively. The data show that the approximate optimum dosage for Compound A is 10 mg/kg and the approximate optimum dosage for donepezil is 0.5 mg/kg.

Intraperitoneal injections of donepezil (at the dose of 0.05 mg/kg; a suboptimal does approximately 1/10 the optimum dose) alone, (+) isopropyl 2-methoxyethyl 4-(2-chloro-3-cyano-phenyl)-1,4-dihydro-2,6-dimethylpyridine-3,5-dicarboxylate [hereinafter Compound A] (at the dose of 1.00 mg/kg; suboptimal does approximately 1/10 the optimum dose) alone, and co-administrations of donepezil (at the dose of 0.05 mg/kg; suboptimal does approximately 1/10 the optimum dose) and Compound A (at the dose of 1.00 mg/kg; suboptimal does approximately 1/10 the optimum dose) are evaluated for enhanced novel object recognition memory in young adult male (3 months old) Sprague-Dawley rats (obtained from Hilltop Lab Animals, Scottdale, PA), with approximate weight ranging from 350 to 450 grams.

The vehicle is 10% Cremophore in 0.9% NaCl; 20% EtOH, 60% PEG 400, and 20% H₂O. The formulations contain 0.05 mg/ml donepezil in the vehicle; and 1.00 mg/ml Compound A in vehicle. The total doses are 0.05 mg/kg and 1.00 mg/kg, respectively.

The rats are divided into four groups which receive the following two injections:
Group 1) Vehicle + Vehicle;
Group 2) donepezil + Vehicle;
Group 3) Vehicle + Compound A;
Group 4) donepezil + Compound A.

All rats receive two i.p, injections: the first injection of the first component is administered 60 minutes prior to training, and the second injection of the second component is administered 30 minutes prior to training.

The data are analyzed by an ANOVA followed by a Tukey-Kramer post-hoc test. Vehicle-Vehicle, donepezil -Vehicle, and Vehicle-Compound A treated rats (n = 6 per group) explore the novel and familiar (previously seen) objects equally (% Exploration = 50%) during the test period that occurred 24 hours after training, indicating that these animals do not remember the familiar object.

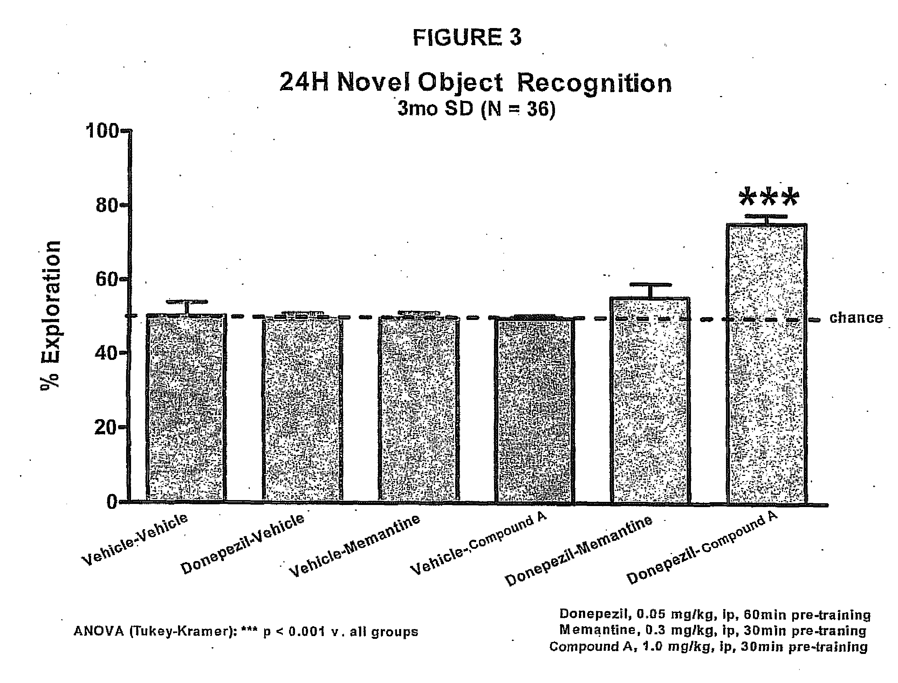

In contrast, rats that receive both donepezil, 0.05 mg/kg and Compound A, 1.00 mg/kg prior to training (n = 6) spend significantly more time (% Exploration >> 50%) exploring the novel object indicating strong memory for the familiar object. This is surprising since the individual suboptimal dosages donepezil, an acetylcholine esterase inhibitor, and Compound A, an L-type calcium channel blocker, are ineffective. All data are shown in the Figure 3.

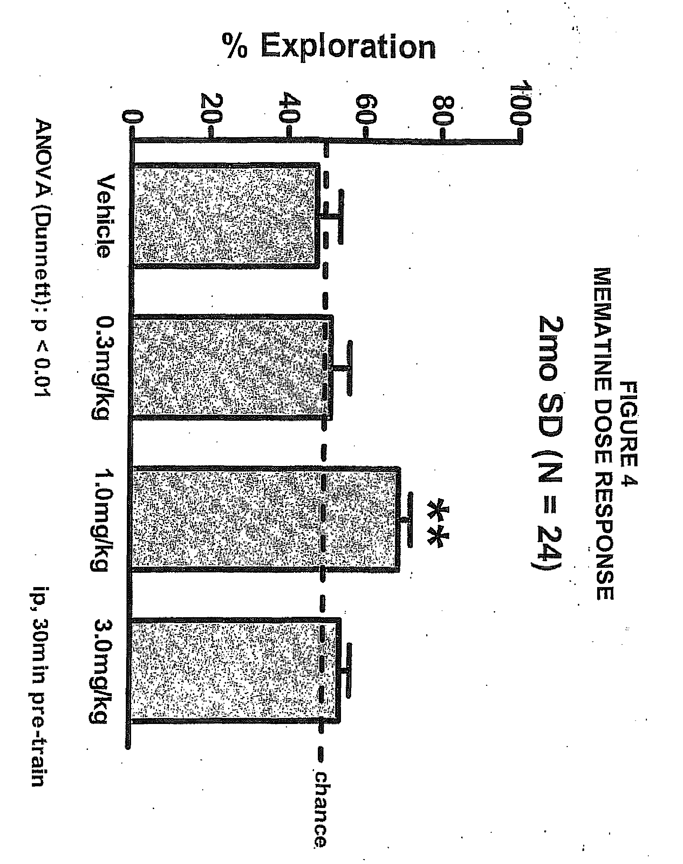

In addition, donepezil, an acetylcholine esterase inhibitor, is tested in combination with the NMDA-R modulator memantine in a similar manner. The dose response data for memantine in the Novel Object Recognition Memory test is shown in Figure 4. The data show that the approximate optimum dosage for memantine is 1.0 mg/kg. Figure 3 presents data for the combination of the suboptimal dose of 0.05 mg/kg donepezil and the suboptimal dose of 0.3 mg/kg of memantine. While the data show a slight improvement in % Exploration in comparison to the controls, Vehicle-Vehicle, donepezil -Vehicle, and Vehicle-memantine, the improvement is much smaller than that shown for donepezil-Compound A.

Memantine and Compound A operate by different mechanisms. Memantine is an NMDA-R modulator whereas Compound A is an L-type calcium channel blocker. Also, donepezil is an acetylcholine esterase inhibitor, and thus acts by a different mechanism than both memantine and Compound A.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.
1. Use of (+)-isopropyl 2-methoxyethyl 4-(2-chloro-3-cyano-phenyl)-1,4- dihydro-2,6-dimetliylpyridine-3,5-dicarboxylate for the production of a medicament for the treatment of memory and/or cognitive impairment, wherein the administration pattern of (+)-isopropyl 2-methoxyethyl 4-(2-chloro-3-cyano-phenyl)-1,4- dihydro-2,6-dimethylpyridine-3,5-dicarboxylate comprises simultaneous or sequential administration with a cholinesterase inhibitor, wherein the cholinesterase inhibitor is selected from the group of rivastigmine, galantamine, huperzine A, and phenserine.
2. (+)-isopropyl 2-methoxyethyl 4-(2-chloro-3-cyano-phenyl)-1,4- dihydro-2,6-dimethylpyridine-3,5-dicarboxylate for the treatment of memory and/or cognitive impairment, wherein the administration pattern of (+)-isopropyl 2-methoxyethyl 4-(2-chloro-3-cyano-phenyl)-1,4- dihydro-2,6-dunethylpyridine-3,5-dicarboxylate comprises simultaneous or sequential administration with a cholinesterase inhibitor, wherein the cholinesterase inhibitor is selected from the group of rivastigmine, galantamine, huperzine A, and phenserine,
3. (+)-isopropyl 2-methoxyethyl 4-(2-chloro-3-cyano-phenyl)-1,4- dihydro-2,6-dimethylpyridine-3,5-dicarboxylate according to claim 2, wherein it is present in a combination with rivastigmine, galantamine, huperzine A, or phenserine.
4. (+)-isopropyl 2-methoxyethyl 4-(2-chloro-3-cyano-phenyl)-1,4- dihydro-2,6-dimethylpyridine-3,5-dicarboxylate according to claim 2 or 3, wherein said memory and/or cognitive impairment is associated with Alzheimer's disease.
5. A pharmaceutical composition comprising (+) isopropyl 2-methoxyethyl 4-(2-chloro-3-cyano-phenyl)-1,4-dihydro-2,6-dimethylpyridine-3,5-dicarboxylate and a cholinesterase inhibitor, wherein the cholinesterase inhibitor is selected from the group of rivastigmine, galantamine, huperzine A, and phenserine.

## Claims

1. Use of (+)-isopropyl 2-methoxyethyl 4-(2-chloro-3-cyano-phenyl)-1,4- dihydro-2,6-dimethylpyridine-3,5-dicarboxylate for the production of a medicament for the treatment of memory and/or cognitive impairment, wherein the administration pattern of (+)-isopropyl 2-methoxyethyl 4-(2-chloro-3-cyano-phenyl)-1,4- dihydro-2,6-dimethylpyridine-3,5-dicarboxylate comprises simultaneous or sequential administration with a cholinesterase inhibitor, wherein the cholinesterase inhibitor is selected from the group of rivastigmine, galantamine, huperzine A, and phenserine.

2. (+)-isopropyl 2-methoxyethyl 4-(2-chloro-3-cyano-phenyl)-1,4- dihydro-2,6-dimethylpyridine-3,5-dicarboxylate for the treatment of memory and/or cognitive impairment, wherein the administration pattern of (+)-isopropyl 2-methoxyethyl 4-(2-chloro-3-cyano-phenyl)-1,4- dihydro-2,6-dimethylpyridine-3,5-dicarboxylate comprises simultaneous or sequential administration with a cholinesterase inhibitor, wherein the cholinesterase inhibitor is selected from the group of rivastigmine, galantamine, huperzine A, and phenserine.

3. (+)-isopropyl 2-methoxyethyl 4-(2-chloro-3-cyano-phenyl)-1,4- dihydro-2,6-dimethylpyridine-3,5-dicarboxylate according to claim 2, wherein it is present in a combination with rivastigmine, galantamine, huperzine A, or phenserine.

4. (+)-isopropyl 2-methoxyethyl 4-(2-chloro-3-cyano-phenyl)-1,4- dihydro-2,6-dimethylpyridine-3,5-dicarboxylate according to claim 2 or 3, wherein said memory and/or cognitive impairment is associated with Alzheimer's disease.

5. (+)-isopropyl 2-methoxyethyl 4-(2-chloro-3-cyano-phenyl)-1,4- dihydro-2,6-dimethylpyridine-3,5-dicarboxylate according to claim 2 or 3, wherein said memory and/or cognitive impairment is associated with dementia.

6. A pharmaceutical composition comprising (+) isopropyl 2-methoxyethyl 4-(2-chloro-3-cyano-phenyl)-1,4-dihydra-2,6-dimethylpyridine-3,5-dicarboxylate and a cholinesterase inhibitor, wherein the cholinesterase inhibitor is selected from the group of rivastigmine, galantamine, huperzine A, and phenserine.
